# EUROPEAN PATENT APPLICATION

(11) **EP 4 282 403 A2**
(43) Date of publication of application: **29.11.2023**
(21) Application number: 22742869.5
(22) Date of filing: 21.01.2022
(51) Int. Cl.: A61K 9/08, A61K 47/32, A61K 47/38, A61K 9/00, A61K 31/196, A61P 27/02, A61P 29/00

(54) **OPHTHALMIC COMPOSITION**

(30) Priority: 21.01.2021 KR 20210008925
(71) Applicant: Taejoon Pharmaceutical Co., Ltd., Seoul 04401 (KR)
(72) Inventor: LEE, Joon Youb, Seoul 04401 (KR); SHIN, Youn Jae, Suwon-si Gyeonggi-do 16281 (KR); YEON, Ji Seon, Suwon-si Gyeonggi-do 16507 (KR)
(74) Representative: HGF
(86) International application number: PCT/KR2022/001095
(87) International publication number: WO 2022/158893

(57) **Abstract**

The present invention relates to an ophthalmic composition with excellent stability.

## Description

### Technical Field

The present invention relates to an ophthalmic composition including bromfenac, and specifically, to an ophthalmic composition including bromfenac, povidone, and hydroxypropylbetadex.

### Background

Inflammatory eye diseases such as conjunctivitis, tarsadenitis, keratitis, blepharitis, scleritis and the like are eye diseases which frequently occur.

Bromfenac is a representative non-steroidal anti-inflammatory drug and exhibits an anti-inflammatory analgesic effect, and thus is advantageously used for treating such inflammatory eye diseases. However, bromfenac may have a stability problem when it is prepared as an aqueous formulation.

Thus, when preparing an ophthalmic composition containing bromfenac, it is essentially required to secure the stability of a preparation.

### [Related Art Reference]

### [Patent Documents]

U.S. Registered Patent Publication No. 4910225

### Detailed Description of the Invention

### Technical Problem

The present invention may provide an ophthalmic composition including bromfenac, a pharmaceutically acceptable salt thereof, a hydrate or solvate thereof, or a mixture thereof; povidone; and hydroxypropylbetadex.

### Technical Solution

The present inventors have made every endeavor to develop novel eye drops in which the stability of a preparation is secured, and as a result, have confirmed that an ophthalmic composition including bromfenac, a pharmaceutically acceptable salt thereof, a hydrate or solvate thereof, or a mixture thereof; povidone; and hydroxypropylbetadex maintains appearance and minimizes the occurrence of related substances during storage to exhibit excellent stability, thereby completing the present invention.

The present invention may provide an ophthalmic composition including bromfenac, a pharmaceutically acceptable salt thereof, a hydrate or solvate thereof, or a mixture thereof; povidone; and hydroxypropylbetadex.

The ophthalmic composition of the present invention may have neither occurrence of insoluble precipitation nor a change in color, maintain a content well, have a less amount of related substances generated during storage to exhibit excellent appearance and storage stability, and may be economical without a need for additional conditions for the improvement of stability. In addition, the ophthalmic composition of the present invention may have an excellent sensation of instillation, and thus may cause little or no foreign body sensation, soreness and the like during instillation, and the composition may be also harmless to the human body, and thus may be advantageously used as an ophthalmic composition.

In the present invention, "bromfenac" may refer to a compound of a following formula, may be a type of acetic acid-based nonsteroidal anti-inflammatory drug (NSAID), and may inhibit cyclooxygenase activity, thereby exhibiting analgesic and anti-inflammatory effects.

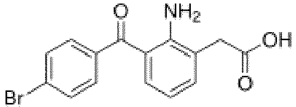

In the present invention, the "pharmaceutically acceptable salt" may refer to a salt commonly used in the pharmaceutical industry, and examples thereof may include inorganic ionic salts such as sodium, etc., inorganic acid salts such as hydrochloric acid, etc., organic acid salts, and the like. For example, in one example, the pharmaceutically acceptable salt may be a sodium salt, but is not limited thereto.

In the present invention, the "hydrate" may refer to one in which bromfenac or a pharmaceutically acceptable salt thereof and water are bound by a non-covalent intermolecular force, and may include a stoichiometric or non-stoichiometric amount of water.

In the present invention, the "solvate" may refer to one in which bromfenac or a pharmaceutically acceptable salt thereof and a solvent other than water are bound by a non-covalent intermolecular force, and may include a stoichiometric or non-stoichiometric amount of the solvent.

In the present invention, bromfenac may refer to a compound represented by above formula, as well as a pharmaceutically acceptable salt thereof, a hydrate or solvate thereof, or a mixture thereof all.

A composition of the present invention may include bromfenac, a pharmaceutically acceptable salt thereof, a hydrate or solvate thereof, or a mixture thereof as an active ingredient, and specifically may include a bromfenac sodium hydrate, but is not limited thereto.

The bromfenac, the pharmaceutically acceptable salt thereof, the hydrate or solvate thereof, or the mixture thereof may be contained in a therapeutically effective amount to achieve a purpose for analgesic and/or anti-inflammatory use, and specifically may be included in an amount of about 0.01 w/v% to about 0.5 w/v%, more specifically about 0.05 w/v% to about 0.5 w/v%, about 0.1 w/v% to about 0.3 w/v% or about 0.05 w/v% to about 0.3 w/v%, and even more specifically about 0.1 w/v% to about 0.2 w/v%, about 0.15 w/v% or about 0.2 w/v% based on the total ophthalmic composition, but is not limited thereto. The bromfenac, the pharmaceutically acceptable salt thereof, the hydrate or solvate thereof, or the mixture thereof may be included in an amount of about 0.01 w/v% or more, about 0.05 w/v% or more, about 0.1 w/v% or more, about 0.15 w/v% or more, or about 0.2 w/v% or more based on the total ophthalmic composition, and may be included in an amount of about 0.5 w/v% or less, about 0.3 w/v% or less, or about 0.2 w/v% or less based on the total ophthalmic composition, but is not limited thereto.

The composition of the present invention may be used for an analgesic and/or anti-inflammatory use due to its property of including bromfenac, a pharmaceutically acceptable salt thereof, a hydrate or solvate thereof, or a mixture thereof, and may be also advantageously used in the prevention or treatment of inflammatory diseases or any symptoms related thereto in an external segment of an eye and an anterior segment of an eye such as blepharitis, conjunctivitis, scleritis including episcleritis, post-operative inflammation, and the like, due to analgesic and/or anti-inflammatory effects.

The composition of the present invention may include povidone (polyvinyl pyrrolidone, PVP).

The povidone may be included in an amount of about 0.1 w/v% to about 10 w/v%, specifically in an amount of about 0.5 w/v% to about 10 w/v% or about 0.5 w/v% to about 5 w/v%, and more specifically in an amount of about 1 w/v% to about 4 w/v%, or about 2 w/v% based on the total ophthalmic composition, but is not limited thereto. The povidone may be included in an amount of about 0.1 w/v% or more, about 0.5 w/v% or more, about 1 w/v% or more based on the total ophthalmic composition, and may be included in an amount of about 10 w/v% or less, about 5 w/v% or less, about 4 w/v% or less based on the total ophthalmic composition, but is not limited thereto.

The composition of the present invention may include hydroxypropylbetadex. Hydroxypropylbetadex may be also called hydroxypropyl-β-cyclodextrin (HP-β-CD).

The hydroxypropylbetadex may be included in an amount of about 0.05 w/v% to about 20 w/v%, specifically in an amount of about 0.1 w/v% to about 20 w/v% or about 0.1 w/v% to about 10 w/v%, and more specifically in an amount of about 0.5 w/v% to about 5 w/v%, about 1 w/v% to about 5 w/v%, or about 2 w/v% based on the total ophthalmic composition, but is not limited thereto. The hydroxypropylbetadex may be included in an amount of about 0.05 w/v% or more, about 0.1 w/v% or more, about 0.5 w/v% or more based on the total ophthalmic composition, and may be included in an amount of about 20 w/v% or less, about 10 w/v% or less, about 5 w/v% or less based on the total ophthalmic composition, but is not limited thereto. It may be preferable that the hydroxypropylbetadex is included in an amount of about 0.1 w/v% or more based on the total ophthalmic composition in terms of stability of the composition of the present invention.

The composition of the present invention may maintain a content and appearance and minimize an amount of related substances generated during storage, thereby exhibiting excellent stability.

The composition of the present invention may include bromfenac, povidone, and hydroxypropylbetadex, and have excellent appearance, content, and storage stability.

In this regard, in one specific example of the present invention, as a result of observing the appearance of the composition of the present invention after storage under stress conditions, it was confirmed that the composition of the present invention shows excellent stability by maintaining the appearance thereof without change during storage, and it was also confirmed that a content thereof is well maintained during storage, and an amount of related substances generated is small during storage, thereby showing excellent stability.

The composition of the present invention may further include additives.

The composition of the present invention may further include at least one additive selected from the group consisting of pH adjusting agents, buffering agents, isotonizing agents, viscosity adjusting agents, solubilizing agents, stabilizing agents, and preservatives.

The pH adjusting agent used herein may include sodium hydroxide, hydrochloric acid, etc., and may be added in an amount required for obtaining an appropriate pH according to a method known to those skilled in the art.

The pH of the composition of the present invention may be about 5 to about 9, specifically about 6 to about 9, but is not limited thereto.

The buffering agent used herein may include acetic acid and/or a salt thereof, citric acid and/or a salt thereof, phosphoric acid and/or a salt thereof, boric acid and/or a salt thereof, borax, and the like, but is not limited thereto. The buffering agent may be included in an amount of about 0.1 w/v% to about 20 w/v% based on the total ophthalmic composition, but is not limited thereto.

The isotonizing agent used herein may include glycerol, mannitol, sorbitol, sodium chloride, potassium chloride, boric acid, etc. The isotonizing agent may be included in an amount of about 1 w/v% to about 20 w/v% based on the total ophthalmic composition, but is not limited thereto.

The viscosity adjusting agent used herein may include a cellulose-based compound including carboxymethylcellulose, etc.; a polyvinyl-based compound including polyvinyl alcohol (PVA), etc.; an acrylic-based compound including carbomer, etc.; a gum compound including xanthan gum, etc.; a polysaccharide including sodium hyaluronate, sodium alginate, etc.; any combination thereof; or the like, and may be added in an amount required for obtaining an appropriate viscosity according to a method known to those skilled in the art. For example, the viscosity adjusting agent may be included in an amount of about 0.05 w/v% to about 10.0 w/v% based on the total ophthalmic composition, but is not limited thereto.

The solubilizing agent used herein may include polyoxyethylene (hydrogenated) castor oil, polyoxyethylene sorbitan fatty acid ester, polyoxyethylene fatty acid ester, a copolymer of polyoxyethylene and polyoxypropylene, polyethylene glycol polyalkylaryl ether, and the like. The solubilizing agent may be included in an amount of about 0.05 w/v% to about 5.0 w/v% based on the total ophthalmic composition, but is not limited thereto.

The stabilizing agent used herein may include sodium edetate, sodium sulfite, etc. The stabilizing agent may be included in an amount of about 0.01 w/v% to about 1.0 w/v% based on the total ophthalmic composition, but is not limited thereto.

The preservative used herein may include: a quaternary ammonium compound including benzalkonium chloride, polyquatemium-1, etc.; a guanidine-based compound including polyhexamethylenebiguanide, chlorohexidine, etc.; chlorobutanol; a mercury-based preservative including thimerosal, etc.; an antioxidant including a stabilized oxychloro complex, p-hydroxybenzoate alkyls, etc.; and the like. The preservative may be included in an amount of about 0.001 w/v% to about 0.02 w/v% based on the total ophthalmic composition, but is not limited thereto.

The composition of the present invention may be specially formulated for topical application, and may be topically administered with formulations such as solutions, emulsions, suspensions, gels, ointments or the like.

The present invention may provide the compositions of (1) to (8) below:
(1) an ophthalmic composition including bromfenac, a pharmaceutically acceptable salt thereof, a hydrate or solvate thereof, or a mixture thereof; povidone; and hydroxypropylbetadex.
(2) the ophthalmic composition of above (1), in which the bromfenac, the pharmaceutically acceptable salt thereof, the hydrate or solvate thereof, or the mixture thereof is a bromfenac sodium hydrate.
(3) the ophthalmic composition of above (1) or (2), in which the bromfenac, the pharmaceutically acceptable salt thereof, the hydrate or solvate thereof, or the mixture thereof is included in an amount of about 0.01 w/v% to about 0.5 w/v% based on the total ophthalmic composition.
(4) the ophthalmic composition of above (1), (2) or (3), in which the povidone is included in an amount of about 0.1 w/v% to about 10.0 w/v% based on the total ophthalmic composition.
(5) the ophthalmic composition of above (1), (2), (3) or (4), in which the hydroxypropylbetadex is included in an amount of about 0.05 w/v% to about 20.0 w/v% based on the total ophthalmic composition.
(6) the ophthalmic composition of above (1), (2), (3), (4) or (5), in which the composition further includes at least one additive selected from the group consisting of pH adjusting agents, buffering agents, isotonizing agents, viscosity adjusting agents, solubilizing agents, stabilizing agents, and preservatives.
(7) the ophthalmic composition of above (1), (2), (3), (4), (5) or (6), in which the composition has a pH of about 5 to about 9.
(8) the ophthalmic composition of above (1), (2), (3), (4), (5), (6) or (7), in which the composition is for anti-inflammation.

The present invention may provide a method for preparing an ophthalmic composition, including mixing bromfenac, a pharmaceutically acceptable salt thereof, a hydrate or solvate thereof, or a mixture thereof; povidone; and hydroxypropylbetadex.

The present invention may provide a method for stabilizing bromfenac, a pharmaceutically acceptable salt thereof, a hydrate or solvate thereof, or a mixture thereof, including mixing bromfenac, the pharmaceutically acceptable salt thereof, the hydrate or solvate thereof, or the mixture thereof; povidone; and hydroxypropylbetadex.

Each component, a content and an effect of the component, and the like mentioned in the preparation method and stabilization method of the present invention may be the same as described above.

Matters mentioned in the ophthalmic composition, the preparation method thereof, and the stabilization method using the same of the present invention may be applied in the same manner, if not contradictory to each other.

The present invention may provide an anti-inflammatory method, including administering the above-described ophthalmic composition into an individual.

For example, the present invention may provide an anti-inflammatory method, including administering an ophthalmic composition containing bromfenac, a pharmaceutically acceptable salt thereof, a hydrate or solvate thereof, or a mixture thereof; povidone; and hydroxypropylbetadex to an individual.

In addition, the present invention may provide a use of the above-described ophthalmic composition for anti-inflammation.

For example, the present invention may provide a use of the ophthalmic composition including bromfenac, a pharmaceutically acceptable salt thereof, a hydrate or solvate thereof, or a mixture thereof; povidone; and hydroxypropylbetadex for anti-inflammation.

In addition, the present invention may provide a use of the above-described ophthalmic composition for the manufacture of an anti-inflammatory medicament.

For example, the present invention may provide a use of the ophthalmic composition including bromfenac, a pharmaceutically acceptable salt thereof, a hydrate or solvate thereof, or a mixture thereof; povidone; and hydroxypropylbetadex for the manufacture of an anti-inflammatory medicament.

In the present invention, the "individual" may refer to all the animals including humans who are likely to develop inflammation.

In the present invention, the "administration" may mean introducing the ophthalmic composition of the present invention into an individual by any appropriate method, and an administration route of the present invention may be to topically administer into eyes due to the nature of the ophthalmic composition. The anti-inflammatory method of the present invention may include administering the ophthalmic composition of the present invention in a therapeutically effective amount.

Each component, a content and an effect of the component, and the like mentioned in the use and anti-inflammatory method of the present invention may be the same as described above.

Matters mentioned in the ophthalmic composition, the preparation method and use thereof, the stabilization method using the same, and the anti-inflammatory method of the present invention may be applied in the same manner, if not contradictory to each other.

### Advantageous Effects

The composition of the present invention may maintain a content and appearance and minimize the occurrence of related substances during storage so as to exhibit excellent stability, and thus can be effectively used as an ophthalmic solution for analgesic and/or anti-inflammatory purposes.

### Brief Description of the Drawings

FIG. 1 is a view confirming the appearance of each composition in Experimental Example 3.
FIG. 2 is a view confirming the precipitates of each composition in Experimental Example 3.
FIG. 3 is a view confirming the appearance of each composition in Experimental Example 4.

### Best Mode for Invention

Hereinafter, the present invention will be described in more detail through examples. These examples are provided only for the purpose of illustrating the present invention, and thus it will be apparent to those skilled in the art that the scope of the present invention is not limited thereto.

### <Experimental Example 1> Stability (1)

Compositions were prepared in accordance with components and contents as shown in table 1 below. Specifically, in Example 1, 2 g of povidone K30 was added to 80 mL of sterile purified water and dissolved, and then 0.5 g of sodium phosphate dibasic anhydrous was added thereto and completely dissolved. 1 g of hydroxypropylbetadex was added and stirred for a certain time to confirm that the hydroxypropylbetadex was completely dissolved, after which 0.15 g of bromfenac sodium hydrate (1.5 hydrate) was added to dissolve and hydrochloric acid was added in an appropriate amount. The total composition was adjusted to 100 mL with sterile purified water, and the composition was prepared through sterile filtration (0.2 µm, membrane filter). The pH of the prepared solution was about 7.8. Comparative Examples 1 to 3 were prepared in the same manner as in Example 1, except for adding povidone and/or HP-β-CD.

**[Table 1]**

| | Example 1 | Comparative Example 1 | Comparative Example 2 | Comparative Example 3 |
|---|---|---|---|---|
| Bromfenac sodium hydrate | 0.15 | 0.15 | 0.15 | 0.15 |
| Povidone | 2.0 | 2.0 | - | - |
| HP-β-CD | 1.0 | - | 1.0 | - |
| Sodium phosphate dibasic anhydrous | 0.5 | 0.5 | 0.5 | 0.5 |
| 1N HCl | q.s. | q.s. | q.s. | q.s. |

| | | | | |
|---|---|---|---|---|
| (Unit: w/v%) | | | | |

The prepared compositions were stored under stress conditions (55°C, 70% RH) for five weeks, and then appearances thereof were observed with the naked eye.

As a result, in the compositions of Comparative Examples 1 and 2, the appearances were changed to orange, and in Comparative Example 3, crystalline precipitation occurred, thus making them difficult to prepare and use as the ophthalmic composition, whereas in the composition of Example 1, the transparent yellow appearance was not changed, and precipitation did not occur. Accordingly, it can be seen that the ophthalmic composition of the present invention has excellent stability.

### <Experimental Example 2> Stability (2)

The compositions of Example 1 and Comparative Examples 1 to 3 were prepared according to above Experimental Example 1. After storing the prepared compositions for five weeks under stress conditions (55°C, 70% RH), the amount of generated 7-(4-bromobenzoyl)indolin-2-one (related substance A) and 2-amino-3-(4-bromobenzoyl)phenyloxoacetic acid (related substance B), which are representative related substances of the bromfenac composition, was measured by liquid chromatography and content (%) thereof was calculated relative to the content of bromfenac. It may be preferable that the content of related substance A is 0.2% or less and that of related substance B is 1% or less relative to that of bromfenac.

As a result, it was found that the compositions of Comparative Examples 1 to 3 have a high content of related substance A or B, whereas the composition of Example 1 has a low content of related substances A and B (Table 2). Accordingly, it can be seen that the ophthalmic composition of the present invention has excellent stability.

**[Table 2]**

| Content (%) | Example 1 | Comparative Example 1 | Comparative Example 2 | Comparative Example 3 |
|---|---|---|---|---|
| Related substance A | 0.04 | 0.03 | 0.41 | 7.87 |
| Related substance B | 0.39 | 3.60 | 0.19 | 0.33 |

### <Experimental Example 3> Stability (3)

Compositions were prepared in accordance with components and contents as shown in table 3 below. Specifically, in Example 2, 4 g of povidone K30 was added to 80 mL of sterile purified water and dissolved, and then 1 g of sodium phosphate dibasic anhydrous was added thereto and completely dissolved. 2 g of hydroxypropylbetadex was added and stirred for a certain time to confirm that the hydroxypropylbetadex was completely dissolved, after which 0.3 g of bromfenac sodium hydrate (1.5 hydrate) was added to dissolve and hydrochloric acid was added in an appropriate amount. The total composition was adjusted to 100 mL with sterile purified water, and the composition was prepared through sterile filtration (0.2 µm, membrane filter). The pH of the prepared solution was about 7.8. Comparative Examples 4 to 6 were prepared in the same manner as in Example 2, except for adding povidone and/or HP-β-CD.

**[Table 3]**

| | Example 2 | Comparative Example 4 | Comparative Example 5 | Comparative Example 6 |
|---|---|---|---|---|
| Bromfenac sodium hydrate | 0.3 | 0.3 | 0.3 | 0.3 |
| Povidone | 4.0 | - | 4.0 | - |
| HP-β-CD | 2.0 | - | - | 2.0 |
| Sodium phosphate dibasic anhydrous | 1.0 | 1.0 | 1.0 | 1.0 |
| 1N HCl | q.s. | q.s. | q.s. | q.s. |

| | | | | |
|---|---|---|---|---|
| (Unit: w/v%) | | | | |

The prepared compositions were stored for 12 days under stress conditions (70°C, 55% RH), after which appearances were observed with the naked eye, and then stored at room temperature and filtered through a cellulose nitrate filter (5.0 µm) to check the occurrence of precipitates.

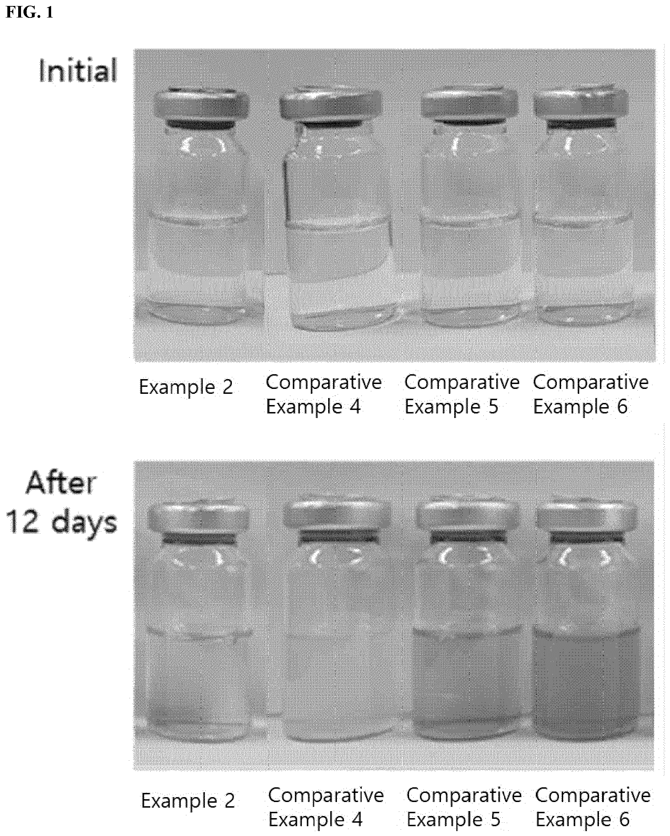

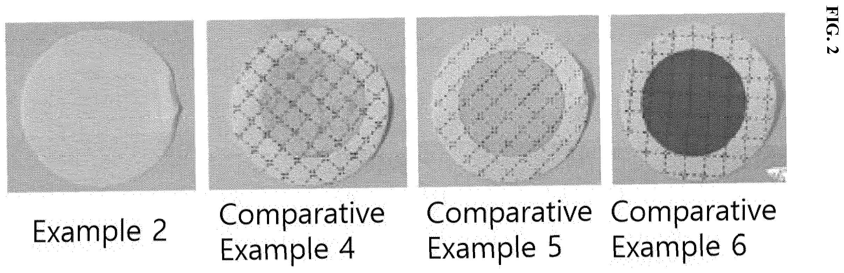

As a result of visual observation, in the composition of Comparative Example 4, crystalline precipitation occurred, and in the compositions of Comparative Examples 5 and 6, appearances were changed to orange, whereas in the composition of Example 2, transparent yellow appearance was not changed and precipitation did not occur (FIG. 1). As a result of confirming whether the precipitates occurred or not, in all the compositions of Comparative Examples 4 to 6, the precipitates were confirmed on filter paper, whereas in the composition of Example 2, the precipitates were not confirmed (FIG. 2). Accordingly, it can be seen that the composition of the present invention has excellent stability.

### <Experimental Example 4> Stability (4)

Compositions were prepared in accordance with components and contents as shown in table 4 below. Specifically, in Example 3, 0.5 g of povidone K30 was added to 80 mL of sterile purified water and dissolved, and then 0.5 g of sodium phosphate dibasic anhydrous was added thereto and completely dissolved. 5 g of hydroxypropylbetadex was added and stirred for a certain time to confirm that the hydroxypropylbetadex was completely dissolved, after which 0.05 g of bromfenac sodium hydrate (1.5 hydrate) was added to dissolve and hydrochloric acid was added in an appropriate amount. The total composition was adjusted to 100 mL with sterile purified water, and the composition was prepared through sterile filtration (0.2 µm, membrane filter). The pH of the prepared solution was about 7.8. Examples 4 and 5 were prepared in the same manner as in Example 3 so that the content of the composition components was the same as described in Table 4.

**[Table 4]**

| | Example 3 | Example 4 | Example 5 |
|---|---|---|---|
| Bromfenac sodium hydrate | 0.05 | 0.05 | 0.5 |
| Povidone | 0.5 | 0.5 | 10 |
| HP-β-CD | 5 | 20 | 5 |
| Sodium phosphate dibasic anhydrous | 0.5 | 0.5 | 0.5 |
| 1N HCl | q.s. | q.s. | q.s. |

| | | | |
|---|---|---|---|
| (Unit: w/v%) | | | |

The prepared compositions were stored under stress conditions (55°C, 70% RH) for four weeks, after which the appearances were observed with the naked eye, and then the content of bromfenac sodium hydrate and the amount of related substances generated were measured by liquid chromatography. The content (%) of the related substances was calculated in the same manner as in Experimental Example 2.

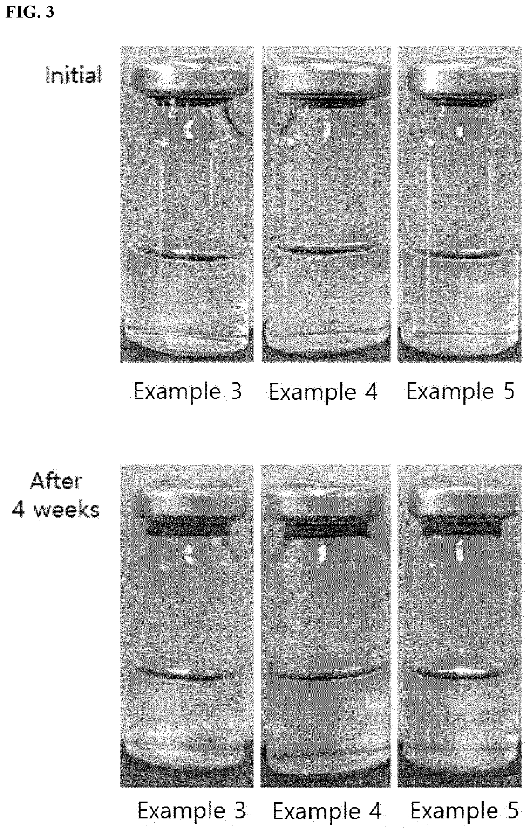

As a result, the appearances of the compositions of Examples 3 to 5 were not changed and precipitation did not occur (FIG. 3), and the content of bromfenac sodium hydrate was also well maintained (Table 5). In addition, it was found that the amount of the related substances generated is also low in all of the compositions of Examples 3 to 5 (Table 5). Accordingly, it can be seen that the composition of the present invention has excellent stability.

**[Table 5]**

| Content (%) | Example 3 | Example 4 | Example 5 |
|---|---|---|---|
| Bromfenac sodium hydrate | 101.9 | 98.9 | 96.5 |
| Related substance A | 0.17 | 0.05 | 0.16 |
| Related substance B | 0.16 | 0.11 | 0.23 |

While specific portions of the present invention have been described in detail above, it is apparent to those skilled in the art that such detailed descriptions are set forth to illustrate examples only, but are not construed to limit the scope of the present invention. Thus, it should be understood that the substantial scope of the present invention is defined by the accompanying claims and equivalents thereto.

## Claims

1. An ophthalmic composition comprising bromfenac, a pharmaceutically acceptable salt thereof, a hydrate or solvate thereof, or a mixture thereof; povidone; and hydroxypropylbetadex.

2. The ophthalmic composition according to claim 1, wherein the bromfenac, the pharmaceutically acceptable salt thereof, the hydrate or solvate thereof, or the mixture thereof is bromfenac sodium hydrate.

3. The ophthalmic composition according to claim 1, wherein the bromfenac, the pharmaceutically acceptable salt thereof, the hydrate or solvate thereof, or the mixture thereof is comprised in an amount of about 0.01 w/v% to about 0.5 w/v% based on the total ophthalmic composition.

4. The ophthalmic composition according to claim 1, wherein the povidone is comprised in an amount of about 0.1 w/v% to about 10.0 w/v% based on the total ophthalmic composition.

5. The ophthalmic composition according to claim 1, wherein the hydroxypropylbetadex is comprised in an amount of about 0.05 w/v% to about 20.0 w/v% based on the total ophthalmic composition.

6. The ophthalmic composition according to claim 1, wherein the composition further comprises at least one additive selected from the group consisting of pH adjusting agents, buffering agents, isotonizing agents, viscosity adjusting agents, solubilizing agents, stabilizing agents, and preservatives.

7. The ophthalmic composition according to claim 1, wherein a pH of the composition is about 5 to about 9.

8. The ophthalmic composition according to claim 1, wherein the composition is for anti-inflammation.

9. An anti-inflammatory method comprising administering the ophthalmic composition according to any one of claims 1 to 7 to an individual.

10. A use of the ophthalmic composition according to any one of claims 1 to 7 for anti-inflammation.

11. A use of the ophthalmic composition according to any one of claims 1 to 7 for the manufacture of an anti-inflammatory medicament.
